# EUROPEAN PATENT SPECIFICATION

(11) **EP 3 897 632 B1**
(45) Date of publication and mention of the grant of the patent: **09.12.2026**
(21) Application number: 19836573.6
(22) Date of filing: 19.12.2019
(51) Int. Cl.: A61K 31/4422, A61K 31/352, A61K 31/366, A61K 31/19, A61P 15/02

(54) **COMPOSITION FOR THE TREATMENT OF FEMALE SEXUAL DYSFUNCTIONS**
ZUSAMMENSETZUNG ZUR BEHANDLUNG WEIBLICHER SEXUELLER DYSFUNKTIONEN
COMPOSITION POUR LE TRAITEMENT DE DYSFONCTIONNEMENTS SEXUELS FÉMININS

(30) Priority: 20.12.2018 IT 201800020416
(43) Date of publication of application: 27.10.2021
(73) Proprietor: Neilos S.r.l., 80063 Piano di Sorrento (NA) (IT)
(72) Inventor: DI MAIO, Umberto, 80063 Piano di Sorrento NA (IT)
(74) Representative: Di Giovine, Paolo
(86) International application number: PCT/IB2019/061082
(87) International publication number: WO 2020/128939

(56) References cited:
- EP-A1- 1 348 439
- EP-A1- 2 322 177
- WO-A1-2010/121081
- WO-A1-2016/056001
- US-A1- 2006 083 724
- US-A1- 2017 266 246
- DI BIASE MANUEL ET AL: "Vulvodynia: Current Etiology, Diagnosis, and Treatment", CURRENT BLADDER DYSFUNCTION REPORTS, SPRINGER US, BOSTON, vol. 11, no. 3, 9 July 2016 (2016-07-09), pages 248 - 257, XP036036531, ISSN: 1931-7212, [retrieved on 20160709], DOI: 10.1007/S11884-016-0381-4
- G MIRANDA VARELLA PEREIRA ET AL: "A systematic review of drug treatment of vulvodynia: evidence of a strong placebo effect", BJOG: AN INTERNATIONAL JOURNAL OF OBSTETRICS AND GYNAECOLOGY, vol. 125, no. 10, 15 April 2018 (2018-04-15), GB, pages 1216 - 1224, XP055539395, ISSN: 1470-0328, DOI: 10.1111/1471-0528.15223

## Description

### FIELD OF THE INVENTION

The present invention relates to a composition for topical use comprising nifedipine, the soy isoflavone Genistein, optionally comprising 18 β-glycyrrhetinic acid, and said composition for its use in the treatment of disorders affecting the female urogenital apparatus such as for example dyspareunia, vulvodynia, vaginism and vaginal atrophy.

### STATE OF ART

The urogenital disorders afflict a huge number of women and have a deep effect on the quality of life. Although several treatments for some of the most serious gynaecological pathologies are available, many urogenital disorders are still little known. Consequently, the treatments are often non-existent, ineffective and/or invasive. Some urogenital disorders are classified as female sexual dysfunctions (FSD) and include a group of disorders of multifactorial and multidimensional origin, involving the onset of a deep discomfort with negative impact on the person's quality of life. About 45% of female population has various disorders defined as FSD. Some thereof affect women in fertile age such as dyspareunia, vulvodynia and vaginism, others appear instead with menopause and in post-menopause age such as vaginal atrophy.

The persistent and recurrent genital pain the woman feels in its vagina or pelvis area during penetration attempts or during the complete vaginal penetration in the sexual intercourse is defined dyspareunia. The pain cause can be anatomical or physiological and not limited to possible lesions of vagina, uterus retroversion, urinary tract infection, lack in lubrication or presence of scar tissue. It can be superficial, introital, when the pain is felt at the vaginal entrance, and in such case it is associated to and/or it causes vulvodynia; or deep: in such case the pain is most frequently caused by endometriosis, by pelvis inflammatory disease or by chronical pelvis pain. The biological causes could be attributable to different problems such as: a) chronical infection from candida, bacterial infections and/or vaginosis relapsing from Gardnerella, vulvar papillomavirus; b) inflammatory: up-regulation of mast cells, in response to infective, chemical, allergic, dystrophic, mechanical factors; c) hormonal causes: vulvovaginal atrophies and dystrophies due to estrogen and/or androgen deficiency involving difficulty in genital excitation at the level of the perivaginal vascular tissues; d) muscle causes: hypertonia up to the tension myalgia of the pelvic floor; e) neurological causes (systemic and peripheral neuropathies including neuropathic pain), connective and immune causes (Sjogren syndrome); f) iatrogenic causes: side effects of perineal and pelvic surgery. Psychosexual and relational causes are associated thereto which can contribute to persistence and increase of the pain feeling, and to the worsening of dyspareunia itself.

Vulvodynia is a pathology affecting at least 6% of women and it can be found at any age. The disease affects dramatically the quality of life, apart from the obvious sexual aspect. The International Society for the Study of Vulvovaginal Disease (ISSVD) has defined vulvodynia as vulvar pain or "vulvar discomfort" lasting at least 3 months, often also defined as neuropathic pain. Most of the time it is described as burning pain, which occurs in absence of a clear identifiable cause or a specific clinically identifiable neurological disorder. It may not be caused by any known factor (spontaneous), in fact rarely it is accompanied by physical trauma or observable pathological conditions, or it can appear in response to a tactile stimulus (caused pain), including a too tight clothing or physical stimulation of the vulvar area, on the occasion of the sexual intercourse or the medical examination. It can be generalized, that is extended to the whole vulvar area, or limited to the vestibular area (then one speaks about "vulvar vestibulitis", VVS), to the clitoris ("clitoralgia"), to the periurethral mucosa or to a limited portion of the vulva. In some scientific studies it is believed that vulvodynia could be correlated to a local chronical inflammation, the cause thereof is attributable to a genetic predisposition (genetic polymorphism) leading to a higher production of TNF-α, IL-1β and reduced production of the receptor for the antagonist of IL-1.

Vaginism is a sexual disorder which appears both at the physical-psychosomatic level and at psychological and emotional level. Vaginism is a condition wherein the involuntary muscle spasms prevent the vaginal penetration, including pelvic exams or the use of tampons. It can be primary or secondary: women with strong aversion or suppression of their own sexuality can have primary vaginism. Secondary vaginism can appear after pelvic reconstructive surgery, vulvar dystrophy or vulvovaginal atrophy. Recently, the classification of the female sexual disorders (FSD, Female Sexual Dysfunctions) processed during the Second Consensus Conference has modified the definitions of the sexual disorders, by eliminating the concept of muscle contraction from vaginism "since there is no sufficient scientific evidence substantiating this statement". However, it is to be underlined that the clinical practice well highlights the reflex defensive contraction of the lifting muscles whenever the woman is afraid that something is introduced in her vagina.

The vaginal atrophy is an inflammation of the vagina which develops when there is a significant decrease in the levels of the female estrogen. The symptoms of the vaginal atrophy include vaginal dryness, dyspareunia, increase in the urinary frequency, infections of the repetitive urinary tract or urinary incontinence. Estradiol, the main estrogen produced by ovaries, performs a fundamental role in keeping the vaginal tissues lubricated and healthy. When the estradiol levels are decreased, the vaginal tissue becomes atrophic-thin, dry and wrinkled. Common conditions characterized by low levels of estrogens causing atrophic vaginitis include menopause, breastfeeding, surgery removal of the ovaries before the menopause natural age. Vagina, vulva, urethra and the bladder trine include all receptors of estrogens and when the levels of estrogens decrease they are subjected to atrophy. Even vulva and vaginal walls become pale and thin and lose their elasticity. This translates in a decrease in the vaginal secretion and sensitivity to trauma and pain. Moreover, the estrogen-deficient vagina develops a level of less acid pH (about 6.8), which increases the probability of infections of the urinary tract. At last, the marked decrease in estrogens has shown to increase the oxidative stress, in particular, to high concentrations, the estrogens tend to have an antioxidant beneficial effect by inhibiting 8-hydroxylation of the bases of DNA of guanine.

The object of the present invention is to provide a composition useful in the treatment of female sexual dysfunctions, in particular for use in the treatment of disorders affecting the female urogenital apparatus such as for example dyspareunia, vulvodynia, vaginism and vaginal atrophy.

The patent application EP2322177 discloses the use of 0.1-1% nitedipine (e.g. in the form of gel, ointment, cream, lotion) for the topical treatment of urogenital dysfunction such as vaginismus.

The patent application US 2006/083724 A1 discloses the use of a composition comprising 0.1-0.3% nifedipine (e.g. cream) for the topical treatment of female sexual dysfunctions, such as pain during intercourse (dyspareunia).

The patent application EP 1 348 439 discloses the use of 0.01-3% soy isoflavones for the topical treatment of vaginal dryness and atrophic vaginitis. The article "AN INTERNATIONAL JOURNAL OF OBSTETRICS AND GYNAECOLOGY,vol. 125,no. 10, 15 April 2018 (2018-04-15), discloses the use of 2% and 4% nitedipine cream for the topical treatment of vulvodynia (dyspareunia).

The international patent application WO 2010/121081 A1 discloses compositions comprising 0.05-3% gylcyrrhetinic acid for the topical treatment of atrophic vaginitis and dyspareunia.

### SUMMARY OF THE INVENTION

The present invention is based upon the search for and identification of a new combination of substances exerting an effective and strengthened action in the treatment of female sexual dysfunctions, in particular for use in the treatment of disorders affecting the female urogenital apparatus such as for example dyspareunia, vulvodynia, vaginism and vaginal atrophy.

The present invention relates to compositions for topical use comprising nifedipine and Genistein and optionally even 18 β-glycyrrhetinic acid and the use thereof in the treatment of female sexual dysfunctions. The present invention allows to obtain at the same time:
- Antioxidant effect
- Anti-inflammatory effect

Other advantages and features of the present invention will result evident from the following detailed description.

### DETAILED DESCRIPTION OF THE INVENTION

The present invention describes a composition comprising as main active ingredients nifedipine and Genistein and optionally even 18 β-glycyrrhetinic acid.

Nifedipine is an antihypertensive drug belonging to the pharmacological class of the calcium antagonists and to the chemical class of 1-4 dihydropiridines.

It acts mainly on the vascular smooth muscle cells by binding to the channels of Ca++ of the plasmatic membrane, by locking the transmembrane flow of cation inside the cell, then by inhibiting the contraction of myocytes and vasoconstriction. Nowadays the calcium antagonist drugs are used in managing the essential hypertension and the angina pectoris.

Within the present invention nifedipine is very interesting thanks to its antioxidant and relaxing power in smooth muscles. In fact, it is known that the oxidative stress condition characterizes pathologies such as vaginal atrophy and endometriosis and that the decrease in the muscle hypertonia can facilitate and make less painful the sexual activity in the patients affected by dyspareunia and vulvodynia.

Several studies were performed to evaluate the antioxidant activity of the calcium antagonists, nifedipine included. For example, in one study the antioxidant effects and the mechanism of antioxidant action of different calcium antagonist drugs, thereamong nifedipine, on the rat's myocardial membrane were evaluated by means of a not enzymatic active oxygen generation system. Nifedipine, however, showed a mild inhibitory effect on the lipidic peroxidation. In another study one evaluated the capability of different calcium antagonists, thereamong nifedipine (tested at the concentration of 100 µM), of preventing peroxidation of the microsomal membranes of the brain isolated by three different areas: hippocampus, frontal cortex and caudate nucleus. The highest antioxidant activity, expressed as inhibition of 50% of peroxidation (IC₅₀ antioxidant) was found for nifedipine (IC₅₀= 4 µM). Moreover, in one clinical study performed in double-blind versus placebo the effectiveness of 2 topical formulations, under the form of cream, including nifedipine at the concentration of 0.2% and 0.4% for the treatment of vulvodynia, was evaluated. Thirty women which have suffered from localized severe vulvodynia for at least 6 months were alternatively assigned to 3 groups of topical treatment (0.2% nifedipine, 0.4% nifedipine and placebo) and they have applied the cream 4 times per day for 6 weeks. The results obtained in the present study have highlighted that the treatment with the formulation including nifedipine (both 0.2% and 0.4%), was able to reduce, even if not significantly, the pain felt during the sexual intercourses.

Considering the antioxidant power and the relaxing activity in the smooth muscles, nifedipine can be considered an active valid drug for implementing products for the topical administration capable of counteracting the vulvar pain. It can further improve the symptoms associated to the female sexual dysfunctions and protect the organism from the oxidative damage.

Isoflavones belong to the category of phytoestrogens, substances of plant origin structurally and functionally similar to the estrogens produced by the organism. Soya (Glycine max) is an annual herbaceous plant belonging to the big family of Legumes (or Papilionacee) originally coming from East Asia the seeds thereof are rich in isoflavones. The most common isoflavones are Genistein, Daidzein and Cumestrol and, of these three compounds, Genistein and Daidzein are particularly abundant in soya.

Genistein is a natural estrogen belonging to the category of isoflavones, of which it represents the most active molecule. Its effectiveness depends upon a chemical structure similar to estradiol, the estrogen produced by the ovary in the fertile phase of the woman's life, which provides to the molecule an action similar to the estrogens thanks to the capability of binding to the same receptor. In particular Genistein binds with high affinity to the receptors for the estrogens of beta type, abundantly present on the skin and in particular in fibroblasts. In literature several actions attributable to Genistein are reported, such as the antioxidant action, antibacterial action, anti-inflammatory action and estrogen-like action.

In a clinical study performed in 2011 on 62 women in climacteric age and in post-menopause the effectiveness of Genistein (75 mcg intravaginal suppositories) was evaluated with respect to the hyaluronic acid on the vaginal atrophy.

A significant improvement in the genital symptomatology, in the colposcopic features and in the vaginal and cervical secretions was noted. In a clinical trial randomized in double blind ended in 2013 and performed on 60 women, the effects of the vaginal administration of a gel based upon soy isoflavones extracted from Glycine max (L.) Merr. (4% vaginal gel, 1g/die, for 12 weeks) for the treatment of the vaginal atrophy were evaluated. At the end of the treatment a decrease in the vaginal dryness and dyspareunia was noted. Moreover, in the group of the treated patients the vaginal pH acidified, going from values equal to 7.1 ± 0.9 (before treatment) to 5.4 ± 0.8 (after treatment). At last, a significant increase in the thickness of epithelium after 12 weeks of treatment was noted.

Moreover, considering that Genistein binds the estrogenic receptors (ER) with higher affinity for ERβ particularly expressed in the neuronal and immune cells, in an in-vivo study its effectiveness in reducing the neuropathic pain associated to the chronical inflammation of the sciatic nerve was evaluated (study performed on rats). To say the truth, the subcutaneous administration of Genistein was effective in reducing the pain in the dose-depending mode. The antioxidant power of Genistein was further found since the anti-nociceptive dose reduced the formation of the reactive species of oxygen and malondialdehyde in the tissues of the injured paw and increased the activity of the antioxidant enzymes. At last, the phytoestrogen showed to have immunomodulatory and anti-inflammatory activities since it reduced NF-κB and the overactivation of pro-inflammatory cytokines. In the light of the several documented beneficial activities, soy isoflavones, in particular Genistein, can be considered a valid functional ingredient for the development of new products for the topical administration capable of counteracting the symptomatology associated to the vaginal atrophy and to the vulvar pain and it can prevent cutaneous aging.

Liquorice (Glycyrrhiza glabra L.) is a perennial herbaceous plant, high as far as one meter, belonging to the family of Fabaceae. One of the main active principles of liquorice is glycyrrhizin, which hydrolyzes in two glucuronic acids and in the relative aglycone, glycyrrhetinic acid. The isomer 18 β of glycyrrhetinic acid, also called enoxolone, is active and known for its anti-inflammatory properties. Among the most important effects which were attributed to 18 β-glycyrrhetinic acid certainly there is the anti-inflammatory one guaranteed by the strong similarity of the compound to endogenous cortisone. The structure of 18 β-glycyrrhetinic acid, in fact, makes it active both versus the glucocorticoid receptors, involved in the inflammation, and mineralcorticoid receptors responsible for the hypertensive effect typical of the liquorice-based preparations. It was demonstrated that the therapeutic doses of pure 18 β-glycyrrhetinic acid do not activate the mineralcorticoid receptors, thus separating the advantages from the unwished effects. Moreover 18 β-glycyrrhetinic acid inhibits some important enzymes metabolizing cortisol, thus prolonging its action without increasing the plasmatic concentration thereof.

In addition, 18 β-glycyrrhetinic acid seems to have also antioxidant activity. In one in-vitro study the antioxidant activity of 18 β-glycyrrhetinic acid and of the synthetic derivatives thereof was evaluated by using a rat liver microsomal NADPH cytochrome P450 reductase system. 18 β-glycyrrhetinic acid showed to be capable of decreasing the formation of oxygen reactive species.

In the light of the lenitive and anti-inflammatory activity, 18 β-glycyrrhetinic acid can be considered an active valid acid for the implementation of products intended to the topical administration capable of counteracting the inflammatory state associated to the pain which characterizes the female sexual dysfunctions.

The inventors have observed that the greatest synergy between the different active ingredients in the treatment of female sexual dysfunctions is obtained with the following concentrations per dosage unit:
nifedipine is in an amount ranging from 1 mg a 10000 mg, one soy isoflavone is in an amount ranging from 1 mg and 10000 mg and 18 β-glycyrrhetinic acid is in an amount ranging from 1 mg e 10000 mg.

The inventors have observed that the greatest synergy between the different active ingredients in the treatment of female sexual dysfunctions is obtained with the following concentrations expressed as percentage by weight:
nifedipine is in a concentration expressed as percentage by weight between 0.001% and 10%, preferably between 0.1 and 5%, one soy isoflavone is in a concentration expressed as percentage by weight between 0.001% and 10%, preferably between 0.1 and 5%, and 18 β-glycyrrhetinic acid is in a concentration expressed as percentage by weight between 0.001% and 10%, preferably between 0.1 and 5%.

The compositions according to the present invention preferably will be formulated for topical use for example as creams, ointments, gels, suspensions, or even liquids suitable for the topical administration. The compositions could include suitable excipients, known to the person skilled in the art and be administered one or more times per day, for one or more weeks, the dosage regime will depend upon the pathology gravity. Such excipients can be selected for example from those usually known in the state of art.

The compositions will be commercialized for example as medical device, medicament, cosmetic. The compositions will be mainly intended to be used by the human beings, but they could be also used on the animals.

The combination of the above-mentioned active ingredients could be used formulated in one single composition according to the various above-described embodiments and prepared for example by mixing the selected active ingredients with possible other excipients as it is known to the person skilled in the art.

### EXAMPLES

Some not limiting examples of the compositions according to the present invention are shown hereinafter. In the examples the percentages are to be meant as percentage by weights. The soy isoflavone according to the claimed invention is Genistein, as used in the examples below.

### EXAMPLE 1

| Active principle | Amount present in formulation |
|---|---|
| Nifedipine | 120 mg |
| Soy isoflavone (ex. Genistein) | 15 mg |
| 18 β-glycyrrhetinic acid | 600 mg |

Pharmaceutical form: cream or emulsion, gel, paste, ointment, solution.

### EXAMPLE 2

| Active principle | % present in formulation |
|---|---|
| Nifedipine | 0.2% |
| Soy isoflavone (ex. Genistein) | 0.1% |
| 18 β-glycyrrhetinic acid | 0.5% |

Pharmaceutical form: cream or emulsion, gel, paste, ointment, solution.

### EXAMPLE 3

| Active principle | Amount present in formulation |
|---|---|
| Nifedipine | 100 mg |
| Soy isoflavone (ex. Genistein) | 60 mg |
| 18 β-glycyrrhetinic acid | 100 mg |

Pharmaceutical form: cream or emulsion, gel, paste, ointment, solution.

### EXAMPLE 4

| Active principle | % present in formulation |
|---|---|
| Nifedipine | 0.1% |
| Soy isoflavone (ex. Genistein) | 0.3% |
| 18 β-glycyrrhetinic acid | 0.2% |

Pharmaceutical form: cream or emulsion, gel, paste, ointment, solution.

### EXAMPLE 5

| Active principle | % present in formulation |
|---|---|
| Nifedipine | 0.5% |
| Soy isoflavone (ex. Genistein) | 1.0% |
| 18 β-glycyrrhetinic acid | 5.0% |

### Experimental data

The authors of the present invention have noted that the administration by topical route of a nifedipine-based cream, one soy isoflavone and optionally 18β-glycyrrhetinic acid on a group of patients with disorders affecting the female urogenital apparatus, in particular dyspareunia, vulvodynia, vaginism and vaginal atrophy showed a significant improvement in the symptomatology with respect to a control group treated with a placebo composition. The improvement was evaluated based upon the standard parameters and tests.

The practice of ovariectomy in rats, that is the removal of both ovaries is actually considered a menopause standard model. Generally, the bilateral removal of the ovaries is performed in young healthy rats in reproductive age, after about 13 weeks of life. Ovariectomy is performed about 15 days before the treatment causing hypoestrogenism and then decrease in the thickness of the vaginal epithelium, vaginal dryness, dyspareunia and vaginal atrophy. The model object is to demonstrate that a topical administration of the present composition induces a significant improvement of the symptomatology by restoring a healthy vaginal environment, normalizing pH, re-generating the vaginal epithelium and increasing natural lubrication of vagina, apart from alleviating symptoms correlated to atrophy such as dryness and pain during the sexual intercourses.

On the contrary, in order to demonstrate the antioxidant effectiveness of the composition of the present invention, particularly interesting in the treatment of disorders affecting the female urogenital apparatus, in-vitro assays result to be suitable such as, for example: DPPH test, radical scavenging activity on the nitric oxide or on the peroxynitrite radical, TEAC (Total radical-trapping antioxidant parameter), FRAP (Ferric reducing-antioxidant power), HORAC (Hydroxyl radical averting capacity), ORAC (Oxygen radical absorbance capacity) tests and the like. Experiments show that a cream based upon nifedipine, one soy isoflavone and optionally 18β-glycyrrhetinic acid results to be effective in the above-described in-vitro and in-vivo tests.

## Claims

1. A composition for topical use comprising nifedipine and at least one soy isoflavone, wherein said isoflavone is Genistein.

2. The composition according to claim 1, further comprising 18 β-glycyrrhetinic acid.

3. The composition according to any one of claims 1 to 2 wherein:
- said nifedipine is in an amount ranging from 1 mg to 10000 mg; and/or
- said soy isoflavone is in an amount ranging from 1 mg to 10000 mg;

4. The composition according to any one of claims 2 to 3 whereinsaid 18 β-glycyrrhetinic acid is in an amount ranging from 1 mg to 10000 mg.

5. The composition according to any one of claims 2 to 4 comprising a plant extract of *Glycyrrhiza glabra L.*

6. The composition according to any one of claims 2 to 5,
wherein:
- said nifedipine is in a concentration by weight between 0.01% and 10%; and/or
- said soy isoflavone is in a concentration by weight between 0.01% and 10%; and/or
- said 18 β-glycyrrhetinic acid is in a concentration by weight between 0.01% and 10%.

7. The composition according to claim 6 in a form selected from cream, emulsion, gel, paste, ointment.

8. The composition according to any one of claims 1 to 7 for use in the treatment of female sexual dysfunctions.

9. The composition according to any one of claims 1 to 8 for use in the treatment of disorders affecting the female urogenital apparatus.

10. The composition according to any one of claims 1 to 9 for use in the treatment of dyspareunia, vulvodynia, vaginism and/or vaginal atrophy.

## Patentansprüche

1. Zusammensetzung zur topischen Anwendung, umfassend Nifedipin und mindestens ein Soja-Isoflavon, wobei das Isoflavon Genistein ist.

2. Zusammensetzung nach Anspruch 1, ferner umfassend 18-β-Glycyrrhetinsäure.

3. Zusammensetzung nach einem der Ansprüche 1 bis 2, wobei:
- das Nifedipin in einer Menge im Bereich von 1 mg bis 10000 mg vorliegt; und/oder
- das Soja-Isoflavon in einer Menge im Bereich von 1 mg bis 10000 mg vorliegt.

4. Zusammensetzung nach einem der Ansprüche 2 bis 3, wobei die 18-β-Glycyrrhetinsäure in einer Menge im Bereich von 1 mg bis 10000 mg vorliegt.

5. Zusammensetzung nach einem der Ansprüche 2 bis 4, umfassend einen Pflanzenextrakt aus *Glycyrrhiza glabra L.*

6. Zusammensetzung nach einem der Ansprüche 2 bis 5, wobei:
- das Nifedipin in einer Gewichtskonzentration zwischen 0,01 % und 10 % vorliegt; und/oder
- das Soja-Isoflavon in einer Gewichtskonzentration zwischen 0,01 % und 10 % vorliegt; und/oder
- die 18-β-Glycyrrhetinsäure in einer Gewichtskonzentration zwischen 0,01 % und 10 % vorliegt.

7. Zusammensetzung nach Anspruch 6 in einer Form, ausgewählt aus Creme, Emulsion, Gel, Paste, Salbe.

8. Zusammensetzung nach einem der Ansprüche 1 bis 7 zur Verwendung bei der Behandlung weiblicher sexueller Dysfunktionen.

9. Zusammensetzung nach einem der Ansprüche 1 bis 8 zur Verwendung bei der Behandlung von Störungen, die sich auf den weiblichen Urogenitalapparat auswirken.

10. Zusammensetzung nach einem der Ansprüche 1 bis 9 zur Verwendung bei der Behandlung von Dyspareunie, Vulvodynie, Vaginismus und/oder vaginaler Atrophie.

## Revendications

1. Composition à usage topique comprenant de la nifédipine et au moins une isoflavone de soja, dans laquelle ladite isoflavone est la génistéine.

2. Composition selon la revendication 1, comprenant en outre de l'acide 18 β-glycyrrhétinique.

3. Composition selon l'une quelconque des revendications 1 à 2, dans laquelle :
- ladite nifédipine est présente en une quantité allant de 1 mg à 10 000 mg ; et/ou
- ladite isoflavone de soja est présente en une quantité allant de 1 mg à 10 000 mg ;

4. Composition selon l'une quelconque des revendications 2 à 3, dans laquelle ledit acide 18 β-glycyrrhétinique est présent en une quantité allant de 1 mg à 10 000 mg.

5. Composition selon l'une quelconque des revendications 2 à 4, comprenant un extrait végétal de *Glycyrrhiza glabra L.*

6. Composition selon l'une quelconque des revendications 2 à 5, dans laquelle :
- ladite nifédipine est présente en une concentration en poids comprise entre 0,01 % et 10 % ; et/ou
- ladite isoflavone de soja est présente en une concentration en poids comprise entre 0,01 % et 10 % ; et/ou
- ledit acide 18 β-glycyrrhétinique est présent en une concentration en poids comprise entre 0,01 % et 10 %.

7. Composition selon la revendication 6 sous une forme choisie parmi crème, émulsion, gel, pâte, pommade.

8. Composition selon l'une quelconque des revendications 1 à 7, pour une utilisation dans le traitement de dysfonctionnements sexuels féminins.

9. Composition selon l'une quelconque des revendications 1 à 8, pour une utilisation dans le traitement de troubles affectant l'appareil urogénital féminin.

10. Composition selon l'une quelconque des revendications 1 à 9, pour une utilisation dans le traitement de dyspareunie, vulvodynie, vaginisme et/ou atrophie vaginale.
